(19) Europäisches Patentamt
European Patent Office
Office européen des brevets

(11) **EP 4 353 829 A1**

(12) **EUROPEAN PATENT APPLICATION**
published in accordance with Art. 153(4) EPC

(43) Date of publication:
**17.04.2024 Bulletin 2024/16**

(21) Application number: **22804694.2**

(22) Date of filing: **17.05.2022**

(51) International Patent Classification (IPC):
**C12Q 1/527** (2006.01)        **G01N 33/52** (2006.01)
**G01N 33/68** (2006.01)

(52) Cooperative Patent Classification (CPC):
**C12Q 1/527; G01N 33/52; G01N 33/68**

(86) International application number:
**PCT/JP2022/020562**

(87) International publication number:
**WO 2022/244783 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.05.2021   JP 2021083432**

(71) Applicants:
• **Renascience Inc.**
  **Tokyo 103-0023 (JP)**

• **Tohoku University**
  **Sendai-shi, Miyagi 980-8577 (JP)**

(72) Inventors:
• **WADA, Yoichi**
  **Sendai-shi, Miyagi 980-8577 (JP)**
• **KURE, Shigeo**
  **Sendai-shi, Miyagi 980-8577 (JP)**

(74) Representative: **Vossius & Partner**
**Patentanwälte Rechtsanwälte mbB**
**Siebertstrasse 3**
**81675 München (DE)**

(54) **METHOD FOR QUANTIFYING PHENYLALANINE IN BLOOD, AND MEASUREMENT KIT USED THEREFOR**

(57)    The present invention provides a method for easily and rapidly quantifying phenylalanine (Phe) contained in blood, and a measurement kit used in the method. The quantification method of the present invention uses a test piece including at least two sheet-shaped members (a first member and a second member), and includes the following steps:

(A) dropping blood, in a state of being separated from the second sheet-shaped member, onto the sample holding layer of the first sheet-shaped member of the test piece, and leaving the blood to stand for a certain period of time;

(B) laminating the first member on the second member such that the sample holding layer and the indicator layer of the second member overlap each other in a non-contact state, dropping a phenylalanine ammonia lyase-containing liquid on the sample holding layer, and leaving the sample holding layer to stand for a certain period of time;

(C) measuring the ammonia concentration from the color of the indicator layer of the second member; and

(D) calculating a blood Phe concentration from the ammonia concentration.

Fig. 1

EP 4 353 829 A1

**Description**

Technical Field

The present invention relates to a method for quantifying the concentration of phenylalanine contained in blood. The present invention also relates to a measurement kit used in the method. Furthermore, the present invention relates to a method for easily confirming and managing a blood phenylalanine concentration of a phenylketonuria patient at a bedside or a place other than a medical institution, using the method or the kit.

Background Art

Phenylketonuria is one of congenital amino acid metabolism abnormalities in which phenylalanine (hereinafter, also referred to as "Phe") cannot be metabolized to tyrosine due to a decrease in the activity of phenylalanine hydroxylase (hereinafter, also referred to as "PAH") in the body, and Phe is accumulated in the body. Significant increase in Phe concentration during infancy leads to severe mental retardation. By controlling the blood Phe concentration within a certain range, mental retardation can be prevented, and normal intellectual development can be acquired. Therefore, a patient with phenylketonuria needs to adjust the intake Phe amount by diet therapy using the blood Phe concentration as a biomarker. Since the blood Phe concentration is susceptible to the amount of Phe contained in the meal and the Phe tolerability depends on the individual's PAH activity, it is necessary to finely adjust the intake Phe amount by repeatedly measuring the blood Phe concentration periodically or non-periodically. For this purpose, it is required that the blood Phe concentration can be easily and quickly measured at the bedside of a patient or in a place other than a medical institution in daily life.

Currently, a method for measuring a blood Phe concentration that can be used in a clinical site is an HPLC method. However, since the method requires at least one week until the result is obtained, it is not suitable as a measurement method in a situation where immediate evaluation is required, such as adjustment of the intake Phe amount in the diet therapy. On the other hand, a method capable of rapid measurement includes a mass spectrometry method used in newborn mass screening. However, since this method requires pretreatment of a specimen and requires a large analytical instrument, it cannot be used for an examination at a place other than a medical institution.

In addition, as a method for diagnosing phenylketonuria, conventionally, a method has been proposed in which Phe labeled with a carbon isotope is administered to a subject orally or by injection, and phenylketonuria is diagnosed by a variation in the amount of the carbon isotope contained in breath (PTL 1), and a method has been proposed in which a dehydrogenase specific to L-phenylalanine in a biological sample, as well as a coenzyme, an electron mediator, and a tetrazolium salt are used as a reaction reagent, formazan is generated by an enzymatic reaction and an oxidation-reduction reaction between the biological sample and the reaction reagent, and L-phenylalanine in the biological sample is quantified by optically or/and electrochemically detecting the formazan ( 2). However, the former method has a problem that a mass analyzer is required for measuring the amount of the carbon isotope. On the other hand, the latter is a method capable of easily and quickly examining phenylketonuria, but is a method capable of simultaneously examining three diseases of galactosemia, maple syrup urine disease, and phenylketonuria, which are congenital metabolic disorders, and is different in principle from the measurement method of the present invention described later.

Citation List

Patent Literature

PTL 1: JPH09-257791A
PTL 2: WO02/018627A

Non-Patent Literature

NPL 1: Journal of Biotechnology 258 (2017) p. 148-157
NPL 2: Clinical practice guideline for neonatal mass screening target diseases 2019, SHINDAN TO CHIRYO SHA, Inc., edited by the Japanese Society for Inherited Metabolic Diseases, page 16
NPL 3: Moat, S. J. et al. J Inherit Metab Dis 43, 179-188 (2020).

2

Summary of Invention

Technical Problem

As mentioned previously, there has been a desire to establish a method for measuring Phe concentration in blood conveniently and quickly. Therefore, an object of the present invention is to provide a method for simply and quickly measuring a blood Phe concentration. Another object of the present invention is to provide a measurement kit used in the method. Further, an object of the present invention is to provide a method for managing a blood Phe concentration in a phenylketonuria patient.

Solution to Problem

The present inventors have paid attention to the fact that when Phe is degraded with phenylalanine ammonia lyase (hereinafter, also referred to as "PAL"), ammonia equivalent to Phe is generated, and considered that the Phe concentration in venous blood can be quantitatively measured using the amount of generated ammonia as an index. However, while conducting studies day and night, there is a problem such that the Phe concentration in venous blood of a subject cannot be accurately measured when capillary blood collected from a fingertip or the like is used as a test sample (test blood), since ammonia contained in sweat or the like is mixed in the blood. Therefore, in order to solve the problem, further intensive studies have been made, and as a result, it has been confirmed that the Phe concentration in venous blood can be accurately measured while capillary blood is used as test blood by once allowing collected capillary blood to coexist with an alkali buffer, to gasify and volatilize ammonium ions contained therein, and then to react with PAL. In addition, even when venous blood is used as the test blood, it is possible to measure the blood Phe concentration of the subject with higher accuracy by gasifying and removing an ammonium ion existing in advance.
The present invention has been completed by accumulating such studies, and has the following embodiments.

(I) Method for quantifying blood Phe concentration

(I-1) A method for quantifying a blood Phe concentration of a subject using a test piece consisting of at least two sheet-shaped members, in which

the test piece is formed by laminating a first sheet-shaped member (hereinafter, this may be abbreviated as a "first member") including a sample holding layer containing an alkali buffer and a second sheet-shaped member (hereinafter, this may be abbreviated as a "second member") including an indicator layer that changes in color by reacting with ammonia gas in a peeled state or a peelable state, and
the above quantification method is a method including the following steps (A) to (D):

(A) a step of dropping blood (test blood) collected from a subject on a sample holding layer of a first member of the test piece as it is or after dilution, in a state of being separated from a second member, and leaving the blood to stand for a certain period of time;
(B) laminating the first member on the second member such that the sample holding layer and the indicator layer of the second member overlap each other in a non-contact state, dropping a PAL-containing liquid on the sample holding layer, and leaving the sample holding layer to stand for a certain period of time;
(C) measuring the ammonia concentration in the test blood from the degree of color of the indicator layer of the second member; and
(D) calculating a blood Phe concentration of the subject from the ammonia concentration.

(I-2) The method for quantifying blood Phe according to (I-1), in which the alkali buffer in the sample holding layer of the first member is an alkali buffer containing boric acid and sodium hydroxide, and
an indicator of the indicator layer of the second member is bromocresol green.
(I-3) The method for quantifying blood Phe according to (I-2), in which

the content of boric acid and sodium hydroxide in the sample holding layer of the first member are 0.40 to 0.45 mg and 0.15 to 0.20 mg, respectively, per member,
the content of bromocresol green in the indicator layer of the second member is 0.3 to 0.5 mg per member,
the amount of the test blood dropped on the sample holding layer in the step (A) is at least 10 $\mu$l,
the amount of PAL in the PAL-containing liquid dropped on the sample holding layer in the step (B) is at least 2 mU, and
the blood Phe concentration that can be calculated in the step (D) is 0 to 1400 $\mu$M, preferably 0 to 437 $\mu$M.

(I-4) The method for quantifying blood phenylalanine according to (I-2) or (I-3), in which the step (C) is a step of measuring absorbance of the indicator layer at a wavelength of 635 nm.
(I-5) The method for quantifying blood Phe according to any one of (I-1) to (I-4), in which the blood (test blood) collected from the subject is capillary blood.
(I-6) The method for quantifying blood Phe according to any one of (I-1) to (I-5), in which the subject is a patient with phenylketonuria.
(I-7) The method for quantifying a blood Phe concentration according to any one of (I-1) to (I-6), in which the method for quantifying a blood Phe is performed using a kit described in (II-1) described later.

These quantification methods can also be referred to as a method for presumptive quantification of a blood Phe or a method for estimating a blood Phe concentration.

(II) Measurement kit for blood phenylalanine

(II-1) A kit containing at least the following (a) and (b) or (a) to (c) for use in the method for quantifying blood Phe according to any one of (I-1) to (I-6) : (a) a test piece obtained by laminating a first member including a sample holding layer containing an alkali buffer and a second member including an indicator layer that changes in color by reacting with ammonia gas in a peeled state or a peelable state;

(b) PAL; and
(c) a solvent.

(III) Method for managing blood Phe concentration

(III-1) A method for managing a blood Phe concentration of a patient with phenylketonuria,
in which the method for quantifying blood Phe according to any one of (I-1) to (I-6) is performed using capillary blood of a phenylketonuria patient as test blood periodically or non-periodically at a place other than a medical institution.
(III-2) The management method according to (III-1), in which the method for quantifying Phe in blood is a method performed using the kit according to (II-1).

Advantageous Effects of Invention

According to the method of the present invention, the blood Phe concentration of a subject can be easily and quickly measured at a place of blood sampling. In addition, according to the method of the present invention, for example, even when a trace amount of blood (capillary blood) from a fingertip is used as the test blood, the Phe concentration in the venous blood or plasma of the subject can be obtained with high accuracy. Therefore, the method of the present invention can be effectively used for screening for early detection of phenylketonuria in a neonate, monitoring of the blood Phe concentration at the bedside of a phenylketonuria patient, and monitoring and management of the blood Phe concentration at a place other than a medical institution, particularly in a dietary therapy in a daily life.

Brief Description of Drawings

Fig. 1 shows a perspective view of an example of the test piece 1.
Fig. 2 is a cross-sectional view taken along line A-A of an example of the test piece 1.
Fig. 3 is a cross-sectional view taken along line B-B of an example of the test piece 1.
Fig. 4 is a view showing a peeling operation between a first sheet-shaped member 2 and a second sheet-shaped member 3 of the test piece 1 and a state in which both are peeled off.
Fig. 5 is a view showing a step (A) of the quantification method of the present invention.
Fig. 6 is a view showing a step (B) of the quantification method of the present invention.
Fig. 7 is a view showing a step (C) of the quantification method of the present invention.
Fig. 8 shows a schematic reaction diagram showing the measurement principle of the present invention.

Fig. 9 shows a cross-sectional view of an AMMONIA TEST KIT II AMICHECK (registered trademark) reaction test paper (AMICHECK) used in an Experimental Example.

Fig. 10 shows a result of measuring the ammonia concentration ($\mu$M) in capillary blood (test blood) collected from a fingertip using the AMICHECK and the ammonia measurement device in Reference Experimental Example 1.

Fig. 11 illustrates a relationship between a time (0 to 10 minutes) (X-axis) during which capillary blood (test blood) is left on the sample holding layer of the spacer part of the AMICHECK after being collected from a fingertip and dropped on the layer, and a measured value of the ammonia concentration in the test blood in Reference Experimental Example 2 (the Y-axis [A.U.] represents "measured value at each leaving time/measured value at resting time of 0 minutes").

Fig. 12 shows the results of adding Phe at a known concentration ($\mu$M) (X-axis) to venous blood (test blood) collected from healthy persons (3 persons: A to C) (test samples (A) to (C)) in Experimental Example 1 and measuring the ammonia concentration ($\mu$M) (Y-axis) in the test samples using the AMICHECK and the ammonia measurement device.

Fig. 13 is a graph showing that, in Experimental Example 2, using capillary blood of a phenylketonuria patient as the test blood, the ammonia concentration ($\mu$M) (Y-axis) in the test blood measured using the AMICHECK and the ammonia measurement device correlates with the plasma Phe concentration of the same patient.

Fig. 14 is a graph showing that, in Experimental Example 3, using capillary blood of a phenylketonuria patient as test blood, the plasma Phe concentration of the same patient predicted from the ammonia value measured by the method of the present invention correlates with the actual plasma Phe concentration of the same patient.

Fig. 15 is a graph plotting the result of experiment A (plasma Phe concentration of PKU patients) measured in Experimental Example 4 (1) on the X-axis and the result of experiment B1 (capillary blood Phe concentration of PKU patients) on the Y-axis, and showing the correlation between both results.

Fig. 16 is a graph plotting the result of experiment B2 (Phe concentration in the test sample) measured in Experimental Example 4 (2) on the X-axis and the result of experiment C2 (ammonia measured value) on the Y-axis, and showing the correlation between the results.

Fig. 17 is a graph plotting the result of experiment A (plasma Phe concentration of PKU patient) on the X-axis in Experimental Example 4 (3) and plotting the plasma Phe concentration estimated by applying the ammonia measured value measured by experiment C1 using the fingertip capillary blood of the PKU patient as the test blood to the regression equation derived from Experimental Examples 4 (1) and (2) on the Y-axis, and showing the correlation between the results.

Description of Embodiments

(I) Method for Quantifying Phe in Blood

A method for quantifying Phe in blood (hereinafter, also referred to as "the quantification method") of the present invention is characterized by performing steps (A) to (D) described later using a test piece (hereinafter, referred to as "the test piece") including at least two sheet-shaped members.

Hereinafter, the test piece and the steps (A) to (D) is described.

(1) the test piece

The test piece includes a first sheet-shaped member (first member) including a sample holding layer containing an alkali buffer, and a second sheet-shaped member (second member) including an indicator layer that changes in color by reacting with ammonia gas. In these members, the first member is disposed (laminated) on the second member. Both members may be in a state of being peeled each other, or may be in a state of being temporarily adhered so as to be peelable so as not to be separated from each other.

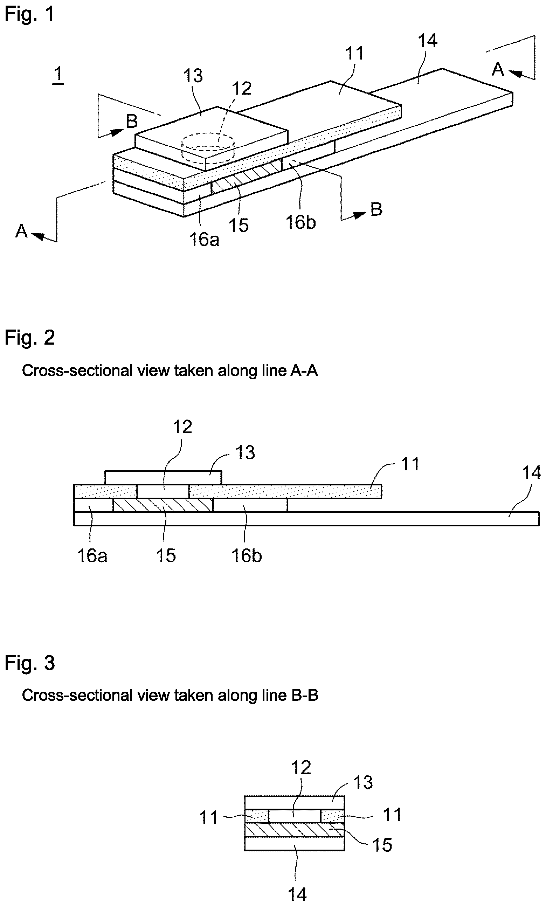

Figs. 1 to 3 shows an example of the test piece 1. Fig. 1 is a perspective view of the test piece 1. Fig. 2 is a cross-sectional view of the test piece 1 shown in Fig. 1 as viewed in the A-A direction. Fig. 3 is a cross-sectional view of the test piece 1 shown in Fig. 1 as viewed in the B-B direction. In these drawings, the same parts are denoted by the same reference numerals.

As illustrated in the drawing, a hole 12 is formed in a sheet-shaped base material 11 (hereinafter, also simply referred to as "first base material 11") of a first member 2 constituting the test piece 1, and a sample holding layer 13 is disposed on the hole so as to cover the hole. On a base material 14 (hereinafter, also simply referred to as "second base material 14") on the sheet of a second member 3 constituting the test piece 1, an indicator layer 15 is disposed in a part corresponding to the hole 12 of the first base material 11, and adhesive layers 16a and 16b are disposed before and after the indicator layer. The hole 12 formed in the first base material 11 is a vent hole for allowing ammonia gas generated in the sample holding layer 13 to reach the indicator layer 15 disposed in the second base material 14 as described later. Therefore, it is also referred to as an $NH_3$ vent hole.

Fig. 1 shows the test piece 1 in a state where the first member 2 is laminated on the second member 3. In this case, as shown in Figs. 2 and 3, the first member 2 is disposed on the second member 3 such that the hole 12 of the first base material 11 is located on the indicator layer 15 of the second member 3, and the opening of the hole 12 is covered with the indicator layer 15. The adhesive layers 16a and 16b in the front and back of the indicator layer 15 are parts for allowing the first member 2 and the second member 3 to be temporarily adhered in a peelable state. The first member 2 and the second member 3 are not necessarily adhered to each other as long as the first member 2 and the second member 3 can be temporarily adhered, and may be in a peeled state each other. The adhesive layers 16a and 16b may be formed so as to be able to repeatedly adhere twice or more. The adhesive layer may be formed only in the front or only in the back of the indicator layer 15.

The size of the first base material 11 is not particularly limited, and in the case of a strip shape, for example, it is 20 to 80 mm length × 3 to 10 mm width × 0.1 to 0.7 mm thickness, preferably 30 to 60 mm length × 4 to 9 mm width × 0.1 to 0.5 mm thickness, and more preferably 30 to 40 mm length × 5 to 8 mm width × 0.1 to 0.4 mm thickness. The size of the second base material 14 is not limited, in the case of a strip shape, preferably has the same width and the different length as the first base material 11. For example, length 20 to 100 mm × width 3 to 10 mm × thickness 0.1 to 0.7 mm, preferably length 40 to 80 mm × width 4 to 9 mm × thickness 0.1 to 0.5 mm, and more preferably length 40 to 60 mm × width 5 to 8 mm × thickness 0.1 to 0.4 mm. The material of these members is also not particularly limited, and examples thereof include polyethylene terephthalate (PET), polyethylene (PE), polypropylene (PP), polymethyl methacrylate (PMMA), polyvinyl chloride (PVC), and polycarbonate (PC). Among them, PET and PC are preferable, and PET is particularly preferable.

The hole 12 formed in the first base material 11 is illustrated as a circular shape in Fig. 1, but is not limited thereto, and may have any shape such as a rectangle. The size of the hole is also not limited, and for example, in the case of a circular shape, the diameter can be set in a range of 2 to 8 mm according to the size (in particular, the width) of the first base material 11. Preferably, it is in the range of 3 to 5.5 mm, more preferably 3.5 to 4.5 mm.

The sample holding layer 13 disposed on the first base material 11 is obtained by impregnating a porous member with an aqueous solution containing an alkali buffer and drying the porous member. As the porous member, any porous member can be used regardless of whether it is fibrous or non-fibrous as long as it is inert to at least blood (whole blood) as a test sample and an alkali buffer and does not affect the method of the present invention. Examples of the fibrous porous member include a filter paper, a nonwoven fabric, and a woven fabric. Examples of the non-fibrous porous member include porous members made of 6-nylon, 6,6-nylon, cellulose acetate, cellulose nitrate, polyethylene, polypropylene, or the like. Although not limited, a non-fibrous porous member is preferable. The size of the sample holding layer 13 is not limited as long as it can fill up the hole 12 formed in the first member 11. For example, as shown in Fig. 1, when the first member 2 has a strip shape, the planar shape of the sample holding layer 13 is preferably rectangular, and the length of one side (width) thereof is preferably the same as the width of the first base material 11. For example, the size is 3 to 10 mm wide × 3 to 10 mm longitudinal × 0.1 to 0.5 mm thickness, preferably 4 to 9 mm wide × 4 to 9 mm longitudinal × 0.1 to 0.4 mm thickness, and more preferably 5 to 8 mm wide × 4 to 9 mm longitudinal × 0.1 to 0.3 mm thickness.

The alkali buffer to be retained in the sample holding layer 13 is not particularly limited as long as it makes the test sample to be spotted to the sample holding layer 13 alkaline and gasifies an ammonium ion contained in the test sample to generate ammonia gas. For example, alkali buffers of pH 9 to 11 such as a borate buffer prepared from boric acid and sodium hydroxide, a sodium borate buffer prepared from sodium borate and sodium hydroxide, and a carbonate-bicarbonate buffer prepared from sodium carbonate and sodium bicarbonate can be exemplified. A borate buffer is preferred. The alkali buffer is dissolved in, for example, ultrapure water to form an alkali buffer, impregnated into the sample holding layer 13, and dried (for example, natural drying, air drying, heat drying, reduced pressure drying, and the like), whereby the sample holding layer 13 can hold the alkali buffer in a dry state.

The indicator layer 15 disposed on the second base material 14 holds an indicator to be described later. The indicator layer 15 may be formed by coating an any member with an indicator, or may be formed by impregnating a porous member with an aqueous solution containing the indicator and drying the porous member, similarly to the sample holding layer 13. These members may be any members that are inert to at least the indicator and ammonia gas and do not affect the method of the present invention. The size of the indicator layer 15 is not limited as long as it can fill up the hole 12 formed in the first base material 11. For example, as shown in Fig. 1, when the second sheet-shaped member 3 has a strip shape, the planar shape of the indicator layer 15 is preferably rectangular, and the length of one side (width) thereof is preferably the same as the width of the second base material 14. For example, the size is 3 to 10 mm wide × 3 to 10 mm longitudinal × 0.01 to 0.3 mm thickness, preferably 4 to 9 mm wide × 4 to 9 mm longitudinal × 0.01 to 0.2 mm thickness, and more preferably 5 to 8 mm wide × 4 to 9 mm longitudinal × 0.01 to 0.1 mm thickness.

As the indicator to be held by the indicator layer 15, a pH indicator having a property of color development or color change in response to a pH change caused by ammonia gas can be used. It may preferably include, but is not limited to, bromocresol green. The bromocresol green is a pH indicator that exhibits a yellow color at a pH of around 3.8 but changes to a blue color at a pH of around 5.4.

In the second member 3, the adhesive layers 16a and 16b disposed in front of and back of the indicator layer 15 are parts for adhering the first member 2 and the second member 3 in a peelable state as described above. The member constituting the adhesive layer, the adhesive component, and the adhering means are not particularly limited as long as they do not affect the method of the present invention. Although not limited, a method using an adhesive such as an acrylic adhesive or an elastomeric adhesive and a method using a double-sided tape can be exemplified as the adhering means, and a lamination method by thermal fusion bonding can also be used. Further, the adhesive layer 16 may be capable of being repeatedly adhered twice or more. In this case, an adhesive or a double-sided tape can be used as the adhering means.

The sizes of the adhesive layers 16a and 16b are also not particularly limited. For example, when the second member 3 has a strip shape, the planar shapes of the adhesive layers 16a and 16b are preferably rectangular, and the length of one side (width) thereof is preferably the same as the width of the second member 14. For example, the size is 3 to 10 mm wide × 2 to 10 mm longitudinal × 0.01 to 0.3 mm thickness, preferably 4 to 9 mm wide × 2 to 9 mm longitudinal × 0.01 to 0.2 mm thickness, and more preferably 5 to 8 mm wide × 2 to 8 mm longitudinal × 0.01 to 0.1 mm thickness. Further, the adhesive layer 16 may be capable of being bonded twice or multiple times.

The test piece 1 can be manufactured by disposing the first member 2 on the second member 3 such that the hole 12 of the first base material 11 is located on the indicator layer 15 of the second member 3 and the opening of the hole 12 is covered with the indicator layer 15, and adhering these members in a peelable state via the adhesive layers 16a and 16b.

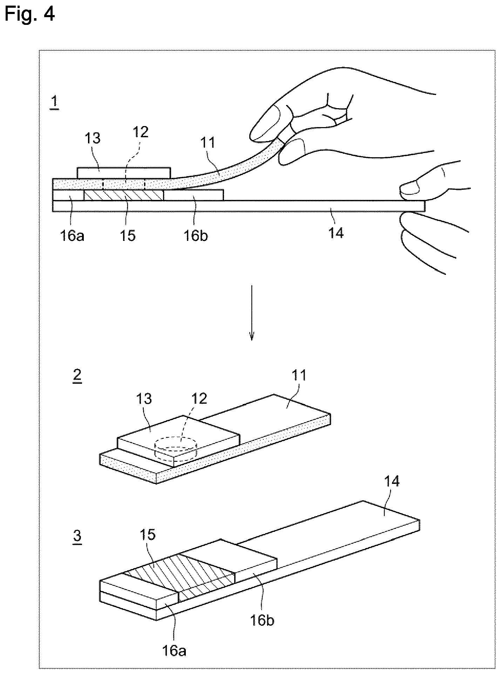

However, as described later, when the quantification method is performed, the test piece 1 is first used in a state where adhesion (temporary adhesion) between the first member 2 and the second member 3 is removed (peeled) and the first member 2 is separated from the second member 3 (see Fig. 4). When the test piece in which the first member 2 and the second member 3 are peeled off from the beginning is used, the peeling operation is unnecessary.

(2) Method for quantifying Phe in blood using the test piece

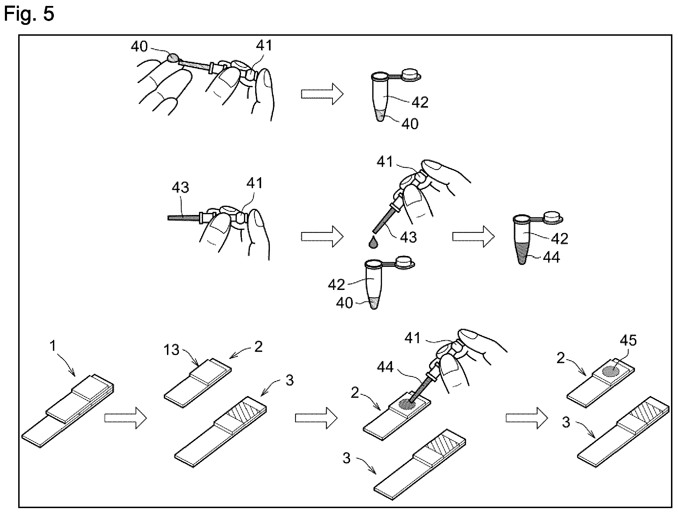

The quantification method using the test piece described above can be performed by the following steps (A) to (D). An example of a series of the steps is shown in Figs. 5 to 7.

(A) dropping blood (test blood) collected from a subject on the sample holding layer 13 of the first member 2 of the test piece 1 in a state of being separated from the second member 3 as it is or after being diluted, and leaving the blood to stand for a certain period of time;
(B) laminating the first member 2 on the second member 3 such that the sample holding layer 13 and the indicator layer 15 of the second member 3 overlap each other in a non-contact state, dropping a PAL-containing liquid onto the sample holding layer, and leaving the sample holding layer 13 to stand for a certain period of time;
(C) measuring the ammonia concentration in the test blood from the degree of color of the indicator layer 15 of the second member 3; and
(D) calculating a blood Phe concentration of the subject from the ammonia concentration.

Step (A) (see Fig. 5)

The step (A) is a step of dropping and spotting the test blood on the sample holding layer of the test piece.
The test blood is blood collected from a subject to be measured. The subject is a neonate suspected of having phenylketonuria (a neonate in need of examination) or a patient with phenylketonuria. The blood is whole blood, and may be capillary blood collected from a capillary, or may be venous blood collected from a vein. Preferably, it is capillary blood. The blood is more preferably capillary blood collected from a fingertip because it is easy to collect blood. The blood may be spotted to the sample holding layer as it is as a test sample, and may be spotted to the sample holding layer after being diluted with a solvent that does not affect the present invention as necessary, for example, when the Phe concentration in the blood is high or the amount of blood collected is small. As an example, but not limited to, one obtained by adding an arbitrary amount of 50 mM Tris-HCl buffer 43 to capillary blood 40 collected from a fingertip and diluting the mixture (diluted sample 44) can also be used as a test sample (see Fig. 5). The dilution rate in the case is not limited, and can be appropriately set so that the ammonia measured value obtained in the step (C) described later falls within the detection range of the blood ammonia measurement device used for the measurement.
The test blood 40 or the diluted sample 44 containing the test blood 40 (also collectively referred to as a "test sample" without distinction) is dropped on the sample holding layer 13 of the first member 2 in a state of being separated from the second member 3 in advance, and is left to stand for 5 minutes or more under room temperature conditions (1 to 30°C, the same applies hereinafter). By the operation, ammonia contained in the test sample can be removed. Specifically, when the test sample is dropped on the sample holding layer 13, the alkali buffer in the sample holding layer is dissolved, the test sample becomes alkaline, and ammonium ions contained in the test sample are gasified. The gasified ammonia gas is volatilized and removed by being left to stand for a certain period of time. The leaving time may be any time as long as ammonia gas can be removed and reduced, and may be, for example, 5 minutes or more under room temperature conditions. Although not limited, it is preferably 8 minutes or more, and more preferably 10 minutes or more. When the rapidity of the result is required, the leaving time is preferably kept within 15 minutes. The time is more preferably about 8 to 10 minutes. Thus, the test sample 45 (deammoniated test sample) from which ammonia has been removed or reduced can be held in the sample holding layer 13 of the first member 2.

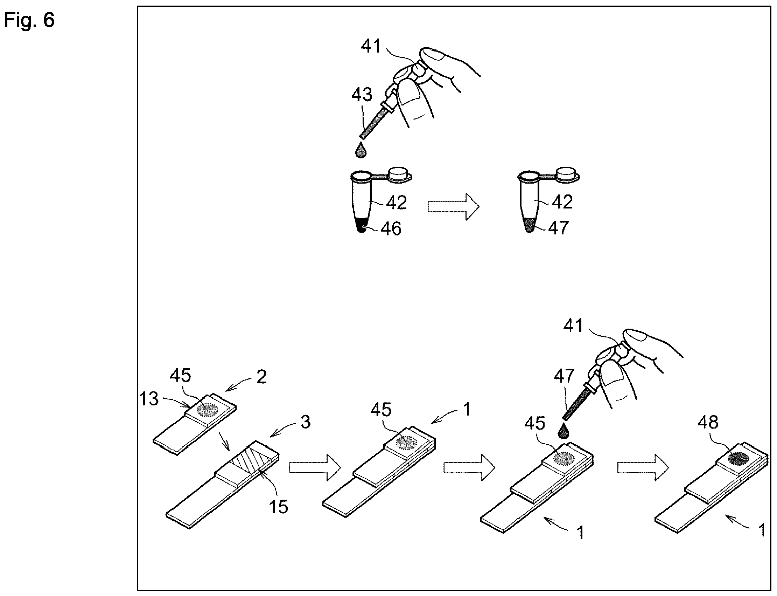

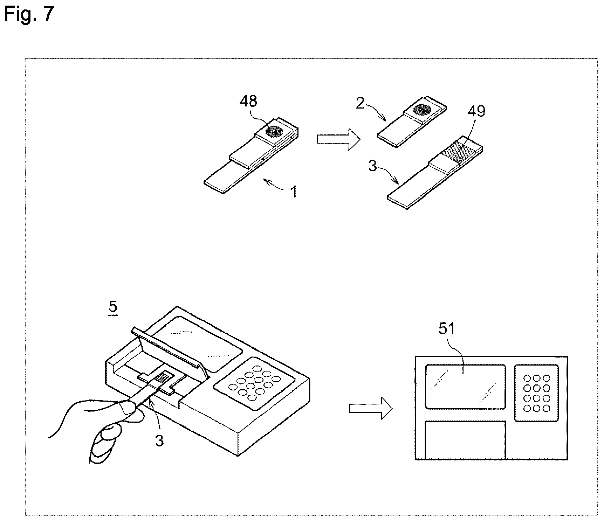

Step (B) (see Fig. 6 and the upper part of Fig. 7)

The step (B) is a step of enzymatically treating the test sample 45 held by the sample holding layer 13 of the first member 2 on the sample holding layer 13.

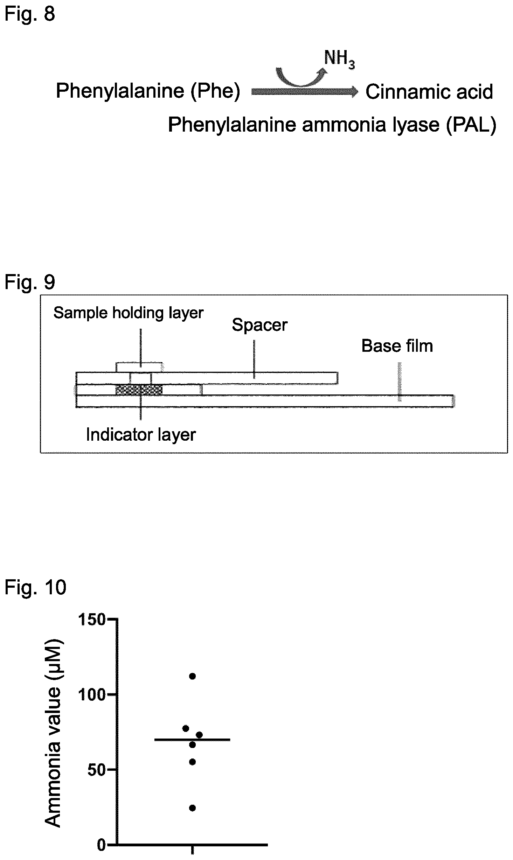

The enzyme used in the present invention is phenylalanine ammonia lyase (PAL). PAL is an enzyme that reacts with Phe to generate equal molar ammonia and cinnamic acid as Phe, as shown in Figure 8. That is, when Phe is contained in the test sample (That is, the test blood), it reacts with PAL to generate equal molar ammonia. PAL can also be prepared using E. coli as shown in Examples described later, but can be commercially obtained conveniently. In particular, as shown in the section of Examples described later, recombinant PAL prepared using E. coli is easy to handle because the specific activity is not significantly reduced even by lyophilization or subsequent storage under room temperature conditions, and the recombinant PAL has good stability.

PAL can be used or stored in a state of being dissolved in a solvent. The solvent is not limited as long as it does not impair the activity of PAL, and for example, a 50 mM Tris-HCl aqueous solution can be exemplified. In addition, as long as the activity of PAL is not impaired, an osmotic pressure regulator such as NaCl, a preservative such as sodium azide, and/or a stabilizer such as trehalose can be blended in addition to the solvent.

(B) The enzyme treatment in the step (A) is performed after the first member 2 obtained in the step (A) is laminated on the second member 3 such that the sample holding layer 13 overlaps the indicator layer 15 of the second member 3 in a non-contact state. When laminated, the opening of the hole 12 of the first base material 11 is covered and filled up by the indicator layer 15 of the second member 3. That is, in the enzyme treatment of the step (B), in a state where the first member 2 is laminated on the second member 3, the PAL-containing liquid 47 obtained by diluting PAL with a solvent is dropped on the sample holding layer 13 containing the test sample 45, and the reaction is carried out by leaving the solution to stand for a certain period of time under room temperature conditions. At this time, the humidity of the environment in which the test piece 1 is left to stand is not limited, but it is preferable to set and adjust the humidity so that the relative humidity falls within the range of 60 to 80% RH. The temperature and humidity during the leaving time may be managed using a thermohygrostat, or may be controlled by placing the test piece in a container or bag including a moisture absorbent such as silica gel.

During the leaving time, Phe derived from the test blood contained in the test sample 45 becomes ammonia by the action of PAL, and the ammonium ion is gasified by the action of the alkali buffer in the sample holding layer 13 to generate ammonia gas. The ammonia gas comes into contact with the indicator layer 15 of the second member 3 through the hole 12 ($NH_3$ vent hole) of the first base material 11. Then, the indicator of the indicator layer 15 reacts and changes in color (color development part 49 of the indicator layer). For example, when the indicator is bromocresol green, the color changes from initial yellow to blue (see the upper part of Fig. 7).

The leaving time may be any time as long as the total amount of Phe contained in the test sample becomes ammonia by PAL, and as a result, the indicator layer 15 changes in color as described above. Under the above temperature and humidity conditions, it may be 5 minutes or more. Although not limited, it is preferably 8 minutes or more, and more preferably 10 minutes or more. When the rapidity of the result is required, the leaving time is preferably kept within 15 minutes. The time is more preferably about 8 to 10 minutes.

Step (C) (see Fig. 7)

The step (C) is a step of calculating the ammonia concentration derived from Phe in the test sample from the color (reference numeral 49) of the indicator layer 15 of the second member 3 obtained in the step (B).

This method can be easily performed by using the blood ammonia measurement device 5. The device is commercially available, and examples thereof include a blood ammonia measurement device PocketChem™ BA PA-4140 manufactured by ARKRAY, Inc.

The measurement device is a small device (measurement principle: 1 wavelength reflection measurement method, measurement wavelength: 635 nm) developed to measure the ammonia concentration in a blood sample using a test paper (AMMONIA TEST KIT II AMICHECK (registered trademark)) sold by the company. The cross-sectional structure of the test paper (referred to as "AMICHECK") is shown in Fig. 9 (incorporated from the attached document of AMICHECK). According to the attached document of AMICHECK, 20 μL of venous blood is dropped on the sample holding layer of AMICHECK, reacted for 180 seconds, then the spacer is peeled off from the base film, and the color development part of the indicator layer of the base film is set in the measurement unit of the blood ammonia measurement device, then the measurement is automatically started, and the ammonia concentration (N-ug/dl) (as the amount of nitrogen) is displayed on the liquid crystal display plate after 20 seconds. The measurement concentration range of ammonia in this apparatus is 6 to 240 $\mu$M, and is 10 to 400 N-$\mu$g/dl in terms of the amount of nitrogen.

In the step (C) of the present invention, the first member 2 and the second member 3 are separated from each other for the test piece 1 subjected to the enzyme treatment in the step (B), and the color development part (49) of the indicator layer 15 of the second member 3 is set in the measurement unit of the blood ammonia measurement device. In this way, the ammonia concentration (N-$\mu$g/dl) (as the amount of nitrogen) is automatically displayed on the liquid crystal display plate. The ammonia concentration reflects the amount of ammonia (Phe-derived ammonia) generated from Phe in the test sample by the action of PAL in the sample holding layer 13 of the first member 2 in the enzyme treatment step (B), and correlates with the amount of Phe contained in the test sample dropped on the sample holding layer 13. When the test blood diluted with the solvent is used as the test sample, the ammonia concentration corresponding to the amount of Phe contained in the test blood can be obtained by performing conversion at the dilution rate.

Step (D)

The step (D) is a step of calculating the blood Phe concentration of the subject from the Phe-derived ammonia concentration contained in the test blood obtained in the step (C). Here, the blood Phe concentration includes both the meaning of the Phe concentration in the venous blood (whole blood) of the subject and the meaning of the Phe concentration in the plasma thereof. It is known that the Phe concentration in whole blood is about 20% less than the Phe concentration in plasma. Therefore, if one Phe concentration can be calculated, the other Phe concentration can be naturally calculated.

The blood Phe concentration of the subject can be calculated from the Phe-derived ammonia concentration in the test sample using a calibration curve prepared in advance by an experiment or a function (regression equation) obtained from the calibration curve.

The calibration curve can be created, for example, as follows according to the origin (venous blood, capillary blood) of the test blood.

When venous blood is used as test blood

(a) The steps (A) to (C) described above are performed on a plurality of test samples prepared by stepwise adding a known amount of Phe to venous blood (whole blood) collected from one healthy person, and the Phe-derived ammonia concentration in the test sample is determined from the degree of color development of the indicator layer of the test piece.

(b) The Phe concentration added to the venous blood is plotted on the horizontal-axis (or vertical-axis), and the Phe-derived ammonia concentration obtained in (a) is plotted on the vertical-axis (or horizontal-axis) to create a calibration curve.

Case of using capillary blood as test blood

(1) The steps (A) to (C) described above are performed using capillary blood (whole blood) collected from fingertips of a plurality of subjects as a test sample, and the Phe-derived ammonia concentration in the test sample is determined from the degree of color development of the indicator layer of the test piece.

(2) Separately, the Phe concentration of the venous blood (whole blood) of the subject is measured by an HPLC method to acquire the blood Phe concentration.

(3) The blood Phe concentration obtained in (2) is plotted on the horizontal-axis (or vertical-axis), and the Phe-derived ammonia concentration obtained in (1) is plotted on the vertical-axis (or horizontal-axis) to create a calibration curve.

The subject is preferably a human having a Phe concentration in venous blood in the range of 0 to 1400 $\mu$M, preferably 0 to 437 $\mu$M, and may be a patient with phenylketonuria or a healthy person who has orally ingested Phe. In addition, both may be mixed in the subjects.

The regression equation can be calculated from these calibration curves.

As shown in Experimental Examples 1 to 4, the Phe-derived ammonia concentration in the test sample obtained in the steps (A) to (C) described above correlates very well with the blood Phe concentration (or plasma Phe concentration) of the subject, and the blood Phe concentration of the subject estimated from the Phe-derived ammonia concentration in the test sample obtained in the steps (A) to (C) (= the blood Phe concentration obtained in the step (D)) almost coincides with the actual blood Phe concentration of the phenylketonuria patient.

Therefore, by using the calibration curve obtained by the above method or the function (regression equation) obtained from the calibration curve, the actual blood Phe concentration of the subject can be estimated with high accuracy from the Phe-derived ammonia concentration in the test sample obtained by performing the steps (A) to (C).

(II) Blood Phe measurement kit

The Phe measurement kit of the present invention contains at least the following (a) and (b), or (a) to (c). By using the kit, the quantification method described above can be easily performed.

(a) A test piece obtained by laminating a first member including a sample holding layer containing an alkali buffer and a second member including an indicator layer that changes in color by reacting with ammonia gas in a peelable state or a peelable state,
(b) PAL, and
(c) a solvent.

(a) The test piece is as described above as the test piece 1, and the above description can be incorporated herein. For convenience, a test paper (AMMONIA TEST KIT II AMICHECK (registered trademark)) manufactured by ARKRAY, Inc. can be used.
(b) From the viewpoint of storage stability, PAL is preferably a lyophilized product. Although not limited, for example, it has been confirmed that recombinant PAL prepared from E. coli have an enzyme activity that is acceptable for use in the present invention for at least 5 weeks even when stored at room temperature in a dried state after lyophilization (see the column of Examples described later).
(c) The solvent can be used for dilution of blood used as test blood and/or solubilization of PAL in lyophilized state. The solvent is not limited; however, for example, a 50 mM Tris-HCl aqueous solution (pH 8.5 to 9.5) or a buffer-containing aqueous solution equivalent thereto can be used.

In addition, the kit of the present invention may include a blood sampling capillary, a pipettor, an Eppendorf (tube), an instruction manual, and the like.

(III) Method for managing blood Phe concentration

By using the quantification method described above, the blood Phe concentration of a phenylketonuria patient can be easily evaluated using venous blood or capillary blood as test blood. The test blood is preferably capillary blood collected from a fingertip. As a result, the blood Phe concentration in a phenylketonuria patient can be managed periodically (for example, every day or at regular intervals) or non-periodically at a place other than a medical institution. Therefore, according to the present invention, it is possible to provide a method for managing the blood Phe concentration in a phenylketonuria patient by using the quantification method described above. The method can be performed using the measurement kit described above.

Examples

Hereinafter, the present invention is described using Experimental Examples in order to help understanding of the configuration and effect of the present invention. However, the present invention is not limited by these Experimental Examples. The following experiments were performed under room temperature (20 $\pm$ 5°C) and atmospheric pressure conditions unless otherwise specified. Unless otherwise specified, in the following, "%" means "% by mass" and "part" means "mass part." In addition, the following experiments were performed after obtaining approval in advance by the Ethics Committee of Tohoku University Graduate School of Medicine (Japan) (approval number 2020-1-362) .

The test piece, the ammonia measurement device, and the PAL-containing solution used in the following Experimental Examples are as follows.

(1) Test piece

AMMONIA TEST KIT II AMICHECK (registered trademark) reaction test paper (ex vivo diagnostics) : manufactured by ARKRAY, Inc.

In the following Experimental Examples, this is referred to as "AMICHECK".

Fig. 9 is a cross-sectional view of AMICHECK. Here, the "spacer" corresponds to the "first base material" of the test piece. As shown in Fig. 9, a hole is formed in the spacer, and a sample holding layer is laminated on an upper surface of the spacer so as to fill up the hole. An alkali buffer (boric acid 0.426 mg/sheet, sodium hydroxide 0.187 mg/sheet) is held in the sample holding layer. In Fig. 9, the "base film" corresponds to the "second base material" of the test piece. An indicator layer is laminated on the base film via a hole of a spacer such that the sample holding layer is laminated in a non-contact state. The indicator layer is coated with bromocresol green (0.04 mg/sheet) as an indicator.

Principle of measurement of ammonia in whole blood by AMICHECK

When a test sample (blood) is spotted to the sample holding layer on the spacer of AMICHECK, the alkali buffer in the sample holding layer dissolves and the test sample becomes alkaline. By this process, ammonium ions in the test sample are gasified to become ammonia gas, and the gas passes through the holes of the spacer and transfers to the indicator layer. When the ammonia gas transferred to the indicator layer reacts with the indicator in the indicator layer, the color of the indicator layer changes. When the spacer laminated on the base film is peeled off and the part of the indicator layer on the base film is subjected to a measurement unit of the following ammonia measurement device, the degree of color change (color intensity) of the indicator layer is automatically sensed and the ammonia concentration (N-$\mu$g/dL) (as the amount of nitrogen) is displayed. In this way, the ammonia concentration in the test sample (blood) can be measured by using the AMICHECK and the ammonia measurement device.

(2) Ammonia measurement device

Blood ammonia measurement device PocketChem™ BA PA-4140:

manufactured by ARKRAY, Inc.
In the following Experimental Examples, this is referred to as an "ammonia measurement device".

(3) PAL-containing liquid

(a) Production of PAL

The cDNA of PAL contained in plasmid (# 78286, Addgene, Cambridge, USA) was inserted into a pET21d(+) vector (69743, Novagen Merck Millipore, Germany) using In-Fusion HD Cloning Kit (Takara, Japan), and a His6 tag was added at the C-terminus. The produced pET21d(+) was transfected into E. coli HMS174(DE3) strain (69453, Merck Millipore, Germany) and cultured until $OD_{600}$ = 0.6, then 1 mM isopropylthio-$\alpha$-D-galactopyranoside (9030, TaKaRa, Japan) was added thereto, and the culture was further continued at 25°C for 15 hours. The cultured E. coli was recovered and lysed using BugBuster protein extraction reagent (70584, Novagen Merck Millipore, Germany), and micrococcal nuclease (2900A, TaKaRa, Japan) was added thereto. PAL was collected with HisSpinTrap (28401353, GE Healthcare, UK) and dialyzed three times (1 hour, 2 hours, 1 day) with 50 mM Tris-HCl solution (pH 8.8) using Mini Dialysis Kit with a 1 kDa cut-off (28955964, GE Healthcare, UK). After concentrating with Amicon Ultra (UFC 500324, Merck Millipore, Germany), the protein concentration was measured with Pierce™ BCA Protein Assay Kit (Thermo Fisher Scientific Inc., Waltham, USA). In addition, the protein was electrophoresed with an SDS-PAGE gel and then stained with TaKaRa CBB Protein Safe Stain (Takara, Japan), and a single band matching the position of the predicted molecular weight was confirmed.

(b) Preparation of PAL-containing liquid

The following three types of PAL-containing liquids were prepared using the recombinant PAL produced using E. coli in the above (a) .

(i) PAL-containing liquid (No. 1)

A PAL-containing liquid is prepared with ultrapure water so as to have the following concentration. Stored at 4°C until use.

| | |
|---|---|
| PAL | 1 to 3 mg/mL (as protein content) |
| Tris-HCl | 50 mM |

(continued)

| | |
|---|---|
| NaCl | 154 mM |
| Sodium azide | 3.3 mM |

(ii) PAL-containing liquid (No. 2)

A PAL-containing liquid (for stock) is prepared in ultrapure water so as to have the following concentration, dispensed in 20 µL parts into an Eppendorf, and stored at -20°C until used. Before use, the product temperature is returned to room temperature, and the volume is made up to 100 µL with 50 mM Tris-HCl to obtain a PAL-containing liquid.

| | |
|---|---|
| PAL | 5 to 15 mg/mL (as protein content) |
| Tris-HCl | 50 mM |
| NaCl | 770 mM |
| Sodium azide | 16.7 mM |

In the Reference Experimental Example and Experimental Example described later, the PAL-containing liquid prepared by the above method was used (the content of PAL in the diluted PAL-containing liquid was 1 to 3 mg/mL (as a protein content)).

(iii) PAL-containing liquid (No. 3)

A PAL-containing liquid (for lyophilization) is prepared so as to have the following concentration, dispensed in 100 µL parts into an Eppendorf, stored at -80°C for 24 hours, and preliminarily frozen. This is lyophilized using a lyophilizer and the resulting lyophilized PAL is stored under room temperature conditions until use. Before use, 100 µL of ultrapure water is added to dissolve the lyophilized PAL to prepare a PAL-containing liquid.

| | |
|---|---|
| Tris-HCl | 50 mM |
| PAL | 1 to 3 mg/mL (as protein content) |
| NaCl | 154 mM |
| Sodium azide | 3.3 mM |
| Trehalose | 10 mg/mL |

(c) Enzyme activity of PAL (specific activity)

Ninety-six (96) µL of a 30 mM aqueous Phe solution was placed in a 96 well plate and allowed to stand at 30°C for 6 minutes, then 4 µL of the PAL-containing liquid (No. 3 before freezing) prepared above was mixed, and the absorbance at 275 nm was measured at 30°C every 5 seconds for 2 minutes. The specific activity (U/mg) was calculated using the following formula (1: Journal of Biotechnology 258 (2017) 148-157).
As a result, the specific activity of PAL (recombinant produced by E. coli) produced in the above (a) was 0.332 ± 0.014 U/mg.

Mathematical formula 1

$$\frac{\Delta A * V * f}{\varepsilon * d * c * v}$$

ΔA: Increasing value of absorbance at 275 nm per minute
V: Total capacity per well (100 µL)
f: Dilution rate of enzyme liquid (1)
ε: Extinction coefficient of cinnamic acid at 275 nm (17.218 mM$^{-1}$cm$^{-1}$)
d: Optical path length (0.28 cm)
c: Enzyme concentration (mg/mL)
v: Dose of enzyme liquid (4 µL)

(d) Stability of PAL

As shown in the following Table 1, even when the PAL is lyophilized and then stored in that state for at least 5 weeks (at room temperature), the PAL retains enzyme activity that is acceptable for use of the present invention.

Table 1 shows the results of measuring the specific activity (U/mg) of the lyophilized PAL prepared in the above (iii) by the method in the above (c) after storing the lyophilized PAL at room temperature for 0 day, 1 week, 3 weeks, and 5 weeks, respectively, and then dissolving 100 μL of ultrapure water into a PAL-containing liquid.

Table 1

| | Before freezing treatment | Storage period after lyophilization treatment | | | |
| --- | --- | --- | --- | --- | --- |
| | | 0 day | 1 week | 3 weeks | 5 weeks |
| Specific activity of PAL (U/mg) | 0.332 ± 0.014 | 0.308 ± 0.014 | 0.198 ± 0.007 | 0.204 ± 0.005 | 0.214 ± 0.007 |

As shown in Table 1, the specific activity of PAL was retained at 0.2 mU or more even when PAL was stored at room temperature for 5 weeks after lyophilization. From this result, it was confirmed that E. coli recombinant PAL is relatively stable in a dried state, and can be used in the quantification method as long as the E. coli recombinant PAL is stored for at least 5 weeks or less under room temperature conditions.

Reference Experimental Example 1

Capillary blood was collected from fingertips of six healthy persons (volunteers) and used as test blood, and the ammonia concentration in the test blood was measured using the AMICHECK and the ammonia measurement device. Measurement of blood ammonia concentration was performed according to the method described in the package insert of AMICHECK.

The results are shown in Fig. 10. The normal blood ammonia value in a healthy person was reported to be 60 μM or less, whereas the average of the obtained ammonia values was as high as 68.20 ± 28.70 μM. In addition, variations in the measured values were observed among the test blood.

As a reason for this, it is considered that ammonia contained in sweat and dirt of the fingertip is mixed when blood is collected from the capillary of the fingertip, and the amount of ammonia in the capillary blood cannot be accurately measured. This is also consistent with the description of "Do not use fingertip blood as it may show a high value if sweat or tissue fluid is mixed." as "operational precautions" in the package insert of AMICHECK.

Reference Experimental Example 2

The method of the present invention is a method for indirectly determining the blood Phe concentration of a subject by measuring the amount of Phe-derived ammonia generated by reacting PAL with Phe of a test sample. For this reason, if the ammonia concentration in the test sample fluctuates before the measurement, the Phe value to be obtained is affected, and the reliability of the Phe value is lowered.

Therefore, as a method for solving this, the following operation was performed using capillary blood collected from fingertips of seven healthy persons (volunteers) as test blood.

(1) The temporarily adhered spacer part (first member) of the AMICHECK and the base film part (second member) are separated.
(2) Ten μL of test blood (whole blood) is dropped on the sample holding layer of the spacer part.
(3) The test blood is left to stand as it is for each of 0 minutes, 1 minute, 2 minutes, 5 minutes, and 10 minutes under the condition of a relative humidity of 40 to 80% RH, then the spacer is superposed on the base film as before, and left to stand as described in the package insert of AMICHECK for 180 seconds, and ammonia gas generated in the sample holding layer is reacted with the indicator layer.
(4) At each leaving time, the indicator layer part of the developed base film is provided to the measurement unit of the ammonia measurement device, and the ammonia measured value displayed on the ammonia measurement device is read.

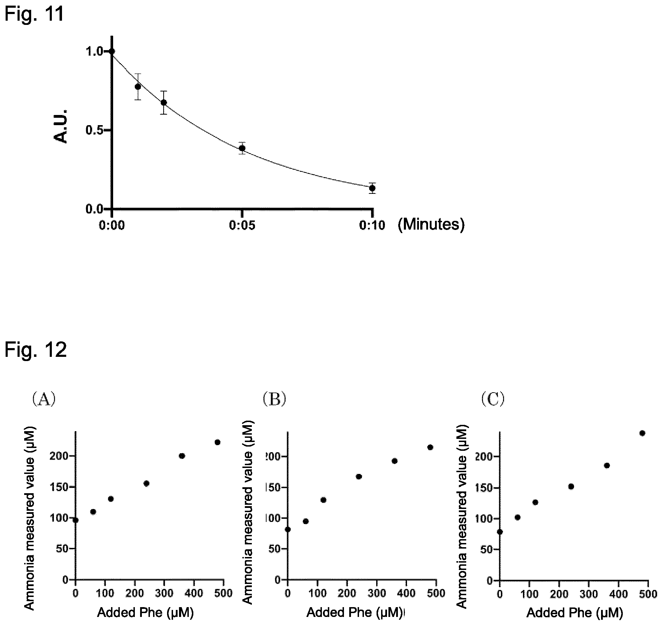

Fig. 11 shows a graph obtained by plotting the leaving time in the step (3) on the X-axis and the ratio between the ammonia measured value for the leaving time of 0 minutes and the ammonia measured value for each leaving time (1 min, 2 min, 5 min, 10 min) (measured value at each time/measured value at 0 min: A.U.) on the Y-axis.

As shown in Fig. 11, it was confirmed that 61.2% of ammonia contained in the test blood was removed by dropping the test blood on the sample holding layer of the spacer and then leaving it to stand for 5 minutes. Furthermore, it was confirmed that up to 86.7% of ammonia contained in the test blood was removed by allowing the test blood to stand for 10 minutes. From this result, it has been confirmed that even if ammonia is contained in the test blood at the concentration of the maximum value measured in Reference Experimental Example 1, 61.2 to 86.7% of the total amount of ammonia is removed and the ammonia content can be reduced to about 14.5 to 26.4 $\mu$M by dropping the test blood on the sample holding layer of the spacer part and then leaving the spacer separated from the base film for a certain period of time such as 5 to 10 minutes. Even if ammonia remains to such an extent in the test blood, there is little influence on the measurement of the blood Phe concentration of the subject.

Experimental Example 1

Blood was collected (venous blood) from veins of three healthy persons (volunteers) (A to C), and a Phe solution having a known concentration was added to and mixed with the test blood at a rate of 5% v/v. The Phe solution was prepared by stepwise dissolving Phe in a 50 mM Tris-HCl aqueous solution so that the concentration of Phe added to the test blood was 0 to 500 $\mu$M.

The test sample obtained by adding and mixing a known amount of Phe to and with the test blood was mixed with 2 times the volume of the PAL-containing liquid, and 20 $\mu$L of the mixture was dropped on the sample holding layer of AMICHECK and left to stand at normal temperature for 10 minutes. Thereafter, the spacer was peeled off from the base film, the indicator layer part on the developed base film is placed on the measurement unit of the ammonia measurement device, and the ammonia measured value displayed on the ammonia measurement device was read.

Fig. 12(A) to Fig. 12(C) show the results of plotting the Phe concentration ($\mu$M) added to the test blood on the X-axis and the measured value ($\mu$M) by the ammonia measurement device on the Y-axis using three venous blood samples as the test blood. As shown in Fig. 12, $R^2$ of the regression line obtained from the results of (A) to (C) was 0.99, 0.97, and 0.99, respectively.

From this result, it was confirmed that Phe in the test sample can be indirectly quantified by measuring the amount of ammonia generated by the reaction with PAL using venous blood containing Phe as the test sample using the AMICHECK.

Experimental Example 2

Ten (10) $\mu$L of capillary blood (test blood) collected from a fingertip of a patient (N = 3) with phenylketonuria (PKU) was dropped on the sample holding layer on the spacer of AMICHECK obtained by separating the spacer (first member) and the base film (second member), and the sample holding layer was left to stand at room temperature for 10 minutes. After being left to stand, the spacer was superposed on the base film at the original position, 10 $\mu$L of the PAL-containing liquid was dropped on the sample holding layer on the spacer, and the spacer was again left to stand at room temperature for 10 minutes. After being left to stand, the indicator layer part on the developed base film was placed on the measurement unit of the ammonia measurement device, and the ammonia measured value displayed on the ammonia measurement device was read.

In parallel, the measurement (HPLC method) of the Phe concentration in blood plasma was entrusted to an external clinical laboratory company (BML, INC.) using the blood plasma of blood (venous blood) collected from the vein of the same PKU patient as a test sample.

HPLC method

Measurement reagent: Amino acid kit (Ninhydrin test solution Wako amino acid automatic analyzer kit: manufactured by FUJIFILM Wako Pure Chemical Corporation)
Amino acid analyzer: L-8900 (Hitachi High-Technologies Corporation)

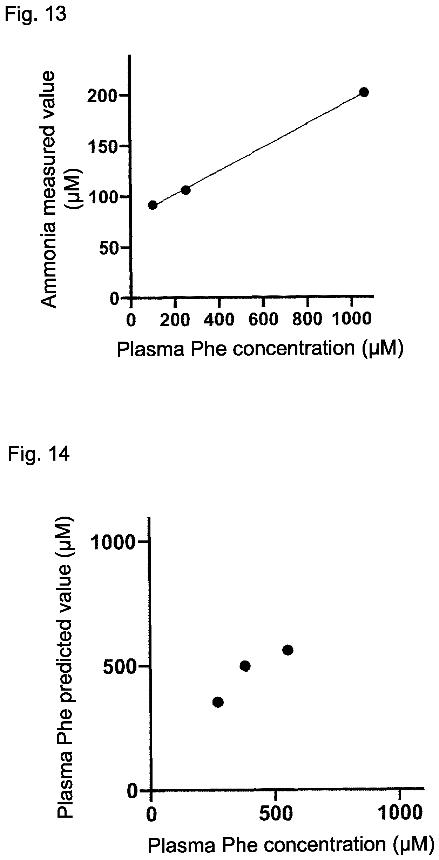

Fig. 13 shows the results obtained by plotting the plasma Phe concentration ($\mu$M) on the X-axis and the ammonia measured value ($\mu$M) by the ammonia measurement device on the Y-axis. As shown in Fig. 13, the regression equation was Y = 0.1151X + 78.78, and $R^2$ was 0.99. From this, the ammonia concentration in the test blood measured by the method of the present invention using the capillary blood of the PKU patient as the test blood and the Phe concentration in the plasma of the same PKU patient correlate very well, and it was confirmed that the possibility that the Phe concentration in the blood or the plasma of the PKU patient can be indirectly calculated by measuring the ammonia concentration in the test blood by the method of the present invention.

Experimental Example 3

In order to verify the results of Experimental Example 2, 10 μL of capillary blood collected from the fingertip of PKU patients (N = 3) different from those of Experimental Example 2 was used as test blood, and the ammonia concentration in the test blood was measured using the AMICHECK and the ammonia measurement device in the same manner as in Experimental Example 2. As conducted in Experimental Example 2, blood (venous blood) collected from the vein of the same PKU patients was used as a test sample, and measurement of the Phe concentration in plasma was outsourced to an external company concurrently.

Fig. 14 shows the results obtained by plotting the value (plasma Phe predicted value) (μM) obtained by converting the ammonia measured value (uM) obtained by using the capillary blood as the test blood into the plasma Phe concentration on the Y-axis and plotting the actually measured plasma Phe concentration (μM) on the X-axis using the regression equation obtained in Experimental Example 2. As illustrated in Fig. 14, $R^2$ obtained from the regression equation was 0.8794, and a good correlation was obtained. From this result, it was confirmed that the blood Phe concentration of the subject can be indirectly calculated by measuring the ammonia concentration in the test blood by the method of the present invention.

When the measurable range of 6 to 240 μM (10 to 400 μg/dL) of the ammonia measurement device is applied to the regression equation obtained in Fig. 13 of Experimental Example 2, the range of detectable blood Phe concentration is estimated to be 0 to 1400 μM.

Experimental Example 4

Experiments A to C described in Table 2 were performed, and the accuracy (correlation between the ammonia concentration in the capillary blood and the plasma Phe concentration) of the quantification method (experiment C) was evaluated by comparing experiment A with experiment B1 and experiment B2 with experiment C, respectively.

Table 2

| Experiment | A | B (B1 and B2) | C (C1 and C2) |
|---|---|---|---|
| Subject | PKU patients | PKU patients (B1) or healthy persons (B2) | PKU patients (C1) or healthy persons (C2) |
| Test blood | Venous blood (plasma) | Capillary Blood | Capillary Blood |
| Measurement target | Phe concentration | Phe concentration | Concentration of $NH_3$ from Phe |
| Measurement method | Conventional Method[1] | Neonatal Mass Screening[2] | Thequantification method[3] |
| Measuring instrument | HPLC | LC-MS/MS | AMICHECK ammonia measurement device |

[1]: Outsourcing (clinical testing company: BML, INC.)
[2]: Outsourcing (clinical testing company: Public Health Society of Miyagi Prefecture)
[LC-MS/MS apparatus] Quadrupole: Xevo TQD (Nihon Waters K.K.)
Liquid feeding device: Binary Solvent Manager (Nihon Waters K.K.)
[Mobile phase] Methanol: Acetonitrile: ultrapure water = 2 : 2 : 1 (formic acid final concentration: 0.05%)
[3]: Fifteen (15) uL of capillary blood was dropped on the sample holding layer on the spacer of AMICHECK from which the base film was separated, and the sample holding layer was allowed to stand at room temperature for 10 minutes. After still standing, the spacer and the base film were returned to their original positions, and 5 μL of a PAL-containing liquid (PAL activity: 2 mU or more per one time) was dropped on the sample holding layer, and the sample holding layer was allowed to stand at room temperature for 10 minutes. After still standing, the color development spot on the base film was measured with the ammonia measurement device.

(1) Experiment A and Experiment B1

Venous blood and capillary blood (blood collected from fingertip) were collected from PKU patients (N = 14), and Phe concentrations in plasma and capillary blood were measured using these blood samples as test blood by the methods of Experimental Examples A and B1 in Table 2, respectively.

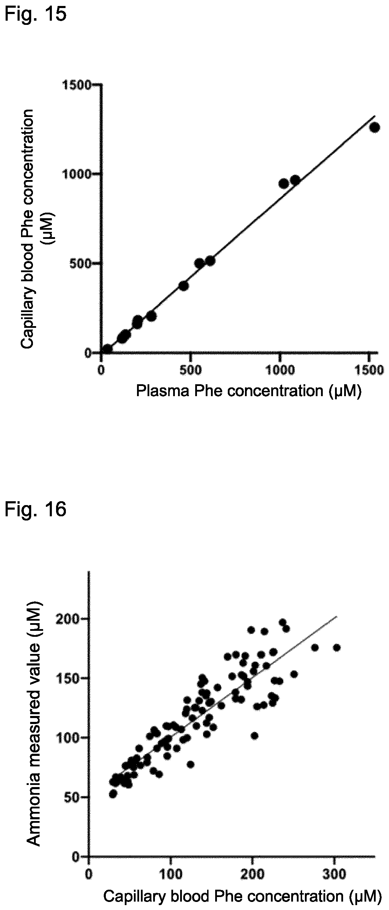

The obtained results were plotted on the X-axis for plasma Phe concentration and on the Y-axis for capillary blood Phe concentration (Fig. 15). The regression equation obtained from the figure was Y = 0.8731X -12.75, and $R^2$ was 0.99. From this result, it can be seen that the plasma Phe concentration and the capillary blood Phe concentration of the PKU patient correlate very well, and the plasma Phe concentration can be estimated from the capillary blood Phe concentration by using the regression equation. It has been previously reported that the plasma Phe concentration and the capillary blood Phe concentration of PKU patients correlate with each other (NPL 3), and the above results are consistent with the previous report.

(2) Experiment B2 and Experiment C2

Each aqueous Phe solution was prepared using ultrapure water as a solvent so that the Phe concentrations were 0 mM, 1.21 mM, 2.42 mM, and 3.63 mM, respectively. An aqueous Phe solution at each concentration was added to and mixed with capillary blood collected from the fingertips of healthy persons at a ratio of 5% v/v, and these were used as test samples (N = 105).

For the test samples, the Phe concentration was measured by the method of Experimental Example B2 in Table 2, and the $NH_3$ concentration was measured by the method of Experimental Example C2.

The obtained results were plotted on the X-axis for the Phe concentration and on the Y-axis for the $NH_3$ concentration (ammonia measured value) (Fig. 16). The regression equation obtained from this figure was Y = 0.5007X + 50.485, and $R^2$ was 0.81.

From this result, it was confirmed that the Phe concentration in the test samples can be indirectly quantified (estimated) by measuring the amount of ammonia (Phe-derived $NH_3$) generated by the reaction with the PAL using the AMICHECK and the ammonia measurement device and applying the regression equation to the obtained ammonia measured value using the capillary blood containing Phe as the test samples.

(3) Discussion of results of experiments A to C

When the two regression equations Y = 0.8731X - 12.75 and Y = 0.5007X + 50.48 obtained in (1) and (2) above are combined, the following relational formula is established:

$$\text{Mathematical formula 2}$$
$$Y = 2.2875X - 100.87$$

Y: Plasma Phe concentration of venous blood
X: Ammonia measured value by the quantification method

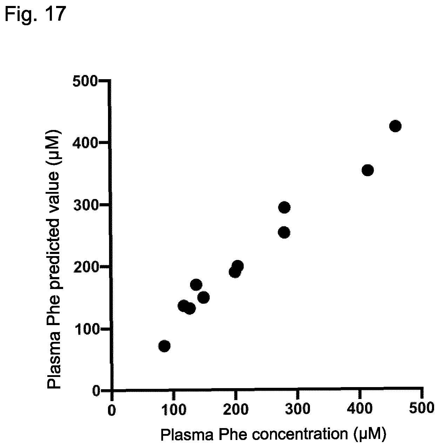

The ammonia measured value obtained by performing the experiment C1 using the capillary blood (N = 11) derived from PKU patients was assigned to X of the relational formula, and the plasma Phe concentration (Y) of the venous blood of the patient was calculated. The value (estimated plasma Phe value) was plotted on the Y-axis, and the plasma Phe concentration of the venous blood of PKU patients obtained in experiment A was plotted on the X-axis (Fig. 17). As illustrated in Fig. 17, $R^2$ of the regression equation was 0.97. The measurement error of the estimated plasma Phe value with respect to the actual plasma Phe value was 9.55% on average (95% confidence interval 4.7 to 14.4), and from this result, it was considered that the quantification (estimated quantification) of the plasma Phe value by the quantification method has accuracy that is acceptable for use in a clinical site or at home.

When the measurable range of 6 to 235 $\mu$M (10 to 400 $\mu$g/dL) of the ammonia measurement device is applied to the regression equation obtained above, the range of the detectable blood Phe concentration is estimated to be 0 to 437 uM.

Industrial applicability

In order to prevent complications in PKU patients, it is recommended to control the plasma Phe concentration within the range of 120 to 360 $\mu$M (Clinical Practice Guideline for Target Disease Subject to Neonatal Mass Screening 2019, SHINDAN TO CHIRYO SHA, Inc., page 16, NPL 2). Therefore, a plasma Phe concentration of 0 to 1400 $\mu$M, preferably 0 to 437 $\mu$M, which can be detected with the method of the present invention, can be sufficiently used clinically. That is, since the method of the present invention is a clinically practical method and can be easily and quickly measured, it can be effectively used for screening for early detection of phenylketonuria in neonates, monitoring of the blood Phe concentration of phenylketonuria patients at the bedside, and monitoring and management of the blood Phe concentration in dietary therapy in daily life.

Description of reference numerals

1 Test piece of present invention
2 First sheet-shaped member of test piece of present invention
3 Second sheet-shaped member of test piece of present invention
11 Base material of first sheet-shaped member (first sheet base material)
12 Hole of first sheet-shaped member ($NH_3$ vent hole)
13 Sample holding layer of first sheet-shaped member
14 Base material of second sheet-shaped member (second sheet base material)
15 Indicator layer of second sheet-shaped member
16a Adhesive layer of second sheet-shaped member (front part of indicator layer)
16b Adhesive layer of second sheet-shaped member (rear part of indicator layer)
40 Test blood
41 Blood collection capillary
42 Eppendorf
43 Solvent
44 Test sample containing test blood and solvent
45 Deammoniated test sample left to stand after dropping on sample holding layer
46 Phenylalanine ammonia lyase (PAL)
47 PAL-containing liquid obtained by dissolving PAL in solvent
48 Reaction spot between test sample containing test blood and PAL-containing liquid
49 Indicator layer developed in response to ammonia gas 5 Ammonia measurement device
51 Display plate indicating measurement result of ammonia measurement device

**Claims**

1. A method for quantifying a blood phenylalanine concentration of a subject using a test piece including at least two sheet-shaped members, wherein

   the test piece is formed by laminating a first sheet-shaped member including a sample holding layer containing an alkali buffer and a second sheet-shaped member including an indicator layer that changes in color by reacting with ammonia gas in a peeled state or a peelable state, and
   the quantification method for blood phenylalanine is a method comprising the following steps (A) to (D):

   (A) dropping blood (test blood) collected from a subject as it is or after dilution on the sample holding layer of the first sheet-shaped member of the test piece in a state of being separated from the second sheet-shaped member, and leaving the blood to stand for a certain period of time;
   (B) laminating the first sheet-shaped member on the second sheet-shaped member such that the sample holding layer and the indicator layer of the second sheet-shaped member overlap each other in a non-contact state, dropping a phenylalanine ammonia lyase-containing liquid on the sample holding layer, and leaving the sample holding layer to stand for a certain period of time;
   (C) calculating the ammonia concentration in the test blood from the degree of color of the indicator layer of the second sheet-shaped member; and
   (D) calculating the blood phenylalanine concentration of the subject from the ammonia concentration.

2. The method for quantifying blood phenylalanine according to claim 1, wherein

   the alkali buffer in the sample holding layer of the first sheet-shaped member is an alkali buffer containing boric acid and sodium hydroxide, and
   an indicator used in the indicator layer of the second sheet-shaped member is bromocresol green.

3. The method for quantifying blood phenylalanine according to claim 2, wherein

   contents of boric acid and sodium hydroxide in the sample holding layer of the first sheet-shaped member are 0.40 to 0.45 mg and 0.15 to 0.20 mg, respectively, per one member, and

a content of bromocresol green in the indicator layer of the second sheet-shaped member is 0.3 to 0.5 mg per one member, and

an amount of test blood applied to the sample holding layer of the first sheet-shaped member in the step (A) is at least 10 $\mu$l, and

an amount of phenylalanine ammonia lyase in the phenylalanine ammonia lyase-containing liquid added dropwise on the sample holding layer in the step (B) is at least 2 mU, and

the blood phenylalanine concentration that can be calculated in the step (D) is 0 to 1400 $\mu$M.

4. The method for quantifying blood phenylalanine according to claim 3, wherein the step (C) is a step of measuring absorbance of the indicator layer at a wavelength of 635 nm.

5. The method for quantifying blood phenylalanine according to claim 1, wherein blood (test blood) collected from the subject is capillary blood.

6. The method for quantifying blood phenylalanine according to claim 1, wherein the subject is a patient with phenylketonuria.

7. A kit for use in the method for quantifying blood phenylalanine according to any one of claims 1 to 6, the kit comprising at least the following (a) and (b) or (a) to (c) :

   (a) a test piece obtained by laminating a first sheet-shaped member including a sample holding layer containing an alkali buffer and a second sheet-shaped member including an indicator layer that changes in color by reacting with ammonia gas in a peeled state or a peelable state;
   (b) phenylalanine ammonia lyase; and
   (c) a solvent.

8. A method for managing a blood phenylalanine concentration in a phenylketonuria patient,
   wherein the method for quantifying a blood phenylalanine concentration according to any one of claims 1 to 6 is performed periodically or non-periodically using capillary blood as a test sample.

9. The management method according to claim 8, wherein the method for quantifying a blood phenylalanine concentration is performed using the kit according to claim 7.

Fig. 1

Fig. 2

Cross-sectional view taken along line A-A

Fig. 3

Cross-sectional view taken along line B-B

Fig. 4

Fig. 5

Fig. 6

Fig. 7

Fig. 8

$$\text{Phenylalanine (Phe)} \xrightarrow[\text{Phenylalanine ammonia lyase (PAL)}]{\quad NH_3 \quad} \text{Cinnamic acid}$$

Fig. 9

Fig. 10

Fig. 11

Fig. 12

(A)

(B)

(C)

Fig. 13

Fig. 14

Fig. 15

Fig. 16

Fig. 17

## INTERNATIONAL SEARCH REPORT

| International application No. |
| --- |
| **PCT/JP2022/020562** |

**A.   CLASSIFICATION OF SUBJECT MATTER**

*C12Q 1/527*(2006.01)i; *G01N 33/52*(2006.01)i; *G01N 33/68*(2006.01)i
FI:   G01N33/68; C12Q1/527; G01N33/52 A

According to International Patent Classification (IPC) or to both national classification and IPC

**B.   FIELDS SEARCHED**

Minimum documentation searched (classification system followed by classification symbols)

C12Q1/527; G01N33/52; G01N33/68

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C.   DOCUMENTS CONSIDERED TO BE RELEVANT**

| Category* | Citation of document, with indication, where appropriate, of the relevant passages | Relevant to claim No. |
| --- | --- | --- |
| A | 血液検査用アンモニアキット　アミチェック, 添付文書, 株式会社アークレイ　ファクトリー-, April 2017<br>  "shape and structure, etc. (kit configuration), "usage and capacity (method of operation)",<br>  (ARKRAY FACTORY, INC.), non-official translation (Ammonia assay kit for blood test:<br>  AMICHECK. Attached document.) | 1-9 |
| A | WO 2006/022113 A1 (TOYAMA PREFECTURE) 02 March 2006 (2006-03-02)<br>  claims 7, 9-10, paragraph [0024] | 1-9 |
| A | JP 58-77663 A (KYOTO DAIICHI KAGAKU KK) 11 May 1983 (1983-05-11)<br>  claim 1 | 1-9 |
| A | 船橋健吾, フェニルケトン尿症診断のための光ファイバ型フェニルアラニン用バイオ<br>センサ, 第60回応用物理学関係連合講演会講演予稿集, 公益社団法人　応用物理学会,<br>11 March 2013, 03-119<br>  entire text, all drawings, (FUNABASHI, Kengo), non-official translation (Optical fiber-bas<br>  ed phenylalanine biosensor for diagnosis phenylketonuria. Lecture preprints of the 60th<br>  JSAP Meeting. The Japan Society of Applied Physics.) | 1-9 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 July 2022** | **26 July 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/020562**

| Patent document<br>cited in search report | Publication date<br>(day/month/year) | Patent family member(s) | Publication date<br>(day/month/year) |
|---|---|---|---|
| WO 2006/022113 A1 | 02 March 2006 | (Family: none) | |
| JP 58-77663 A | 11 May 1983 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H09257791 A

- WO 02018627 A

**Non-patent literature cited in the description**

- *Journal of Biotechnology,* 2017, vol. 258, 148-157
- Clinical practice guideline for neonatal mass screening target diseases 2019. SHINDAN TO CHIRYO SHA, Inc, 16

- **MOAT, S. J. et al.** *J Inherit Metab Dis,* 2020, vol. 43, 179-188
- Clinical Practice Guideline for Target Disease Subject to Neonatal Mass Screening 2019. SHINDAN TO CHIRYO SHA, Inc, 16